# EUROPÄISCHE PATENTANMELDUNG

(11) **EP 3 750 471 A1**
(43) Veröffentlichungstag der Anmeldung: **16.12.2020**
(21) Anmeldenummer: 20175329.0
(22) Anmeldetag: 19.05.2020
(51) Int. Cl.: A61B 3/028, A61B 3/032, A61B 3/00, A61B 3/04

(54) **VERFAHREN ZUM ÜBERPRÜFEN VON AUGEN UND SEHPRÜFSYSTEM**

(30) Priorität: 12.06.2019 DE 102019115929
(71) Anmelder: Oculus Optikgeräte GmbH, 35582 Wetzlar (DE)
(72) Erfinder: DEGLE, Stephan, 07743 Jena (DE)
(74) Vertreter: advotec.

(57) **Zusammenfassung**

Die Erfindung betrifft ein Verfahren zum Überprüfen von Augen eines Probanden mit einem Sehprüfsystem (10) sowie ein Sehprüfsystem, wobei vor den Augen eine Messbrille des Sehprüfsystems mit zumindest einem Probeglas angeordnet wird, wobei mittels einer Kamera (14) einer Kameravorrichtung (13) eines Anzeigegeräts (11) zumindest ein numerischer Wert eines das Probeglas charakterisierenden optischen Parameters erfasst wird, wobei mittels einer Steuervorrichtung des Sehprüfsystems das Anzeigegerät gesteuert wird, wobei der numerische Wert von der Steuervorrichtung mittels Bildverarbeitung erkannt wird, wobei der numerische Wert für eine Sphäre und/oder einen Zylinder und/oder eine Achse und/oder ein Prisma erkannt wird.

## Beschreibung

Die Erfindung betrifft ein Verfahren zum Überprüfen von Augen eines Probanden mit einem Sehprüfsystem sowie ein Sehprüfsystem, wobei vor den Augen eine Messbrille des Sehprüfsystems mit zumindest einem Probeglas angeordnet wird.

Derartige Sehprüfsysteme sind aus dem Stand der Technik hinlänglich bekannt und werden regelmäßig zu einer Durchführung von Sehtests verwendet. So verfügen die bekannten Sehprüfsysteme über ein Anzeigegerät mit einem Bildschirm, mittels dessen einem Probanden Sehprüfzeichen visualisiert bzw. dargeboten werden können. Auch kann ein derartiges Sehprüfsystem ein Steuergerät umfassen, über welches eine Bedienperson eine Wiedergabe von Sehprüfzeichen auf dem Bildschirm steuern kann. Je nach Art des Bildschirms kann dieser auch über eine lineare oder zirkulare Polarisation verfügen. Die Polarisation des Bildschirms wird regelmäßig zur Durchführung von Sehtests in Verbindung mit einer Messbrille oder einem Phoropter genutzt, welche bzw. welcher vor den Augen des Probanden angeordnet wird. Weiter kann das Sehprüfsystem eine Kameravorrichtung mit einer Kamera umfassen, welche beispielsweise zur Vermessung der Augen des Probanden genutzt werden kann. Eine Bestimmung einer subjektiven Refraktion der Augen des Probanden erfolgt durch eine Darbietung verschiedenster Sehprüfzeichen durch eine Bedienperson des Sehprüfsystems in Verbindung mit Probegläsern einer Messbrille oder eines Phoropters. Dabei können auch verschiedene, allgemein bekannte Sehtests, unter anderem eine Testung eines Nahsehens und eines Fernsehens, mit dem Sehprüfsystem durchgeführt werden. Ein derartiges Sehprüfsystem wird beispielsweise von der EP 3 192 432 A2 offenbart.

Weiter geht aus der EP 3 178 376 A1 sowie der EP 3 179 296 A1 jeweils eine Messbrille zur Bestimmung einer subjektiven Refraktion eines Probanden hervor, welche in Kombination mit einem Sehprüfsystem zum Einsatz kommt. Diese Messbrille weißt zwei Glashaltevorrichtungen zur Aufnahme von Probegläsern bzw. Einsteckgläsern, eine Brücke, die die Glashaltevorrichtungen relativ zueinander in einem Abstand verstellbar verbindet, eine Nasenauflagevorrichtung, mit einer verschwenkbaren und höhenverstellbaren Nasenauflage, sowie zwei Bügel auf, die längenveränderbar und höhenverstellbar ausgebildet sind. Weiter weisen die Glashaltevorrichtungen mehrere Glasaufnahmen auf, welche jeweils zumindest einen Andruckfinger zum Klemmen eines Probeglases bzw. Einsteckglases umfassen. Die Glasaufnahmen können jeweils aus zumindest zwei oder drei Stegen ausgebildet sein, die Probegläser bzw. Einsteckgläser an deren Umfang haltern können. Somit können gleichzeitig eine Reihe von Probegläsern, beispielsweise zur Korrektur von Kurzsichtigkeit, Weitsichtigkeit und Astigmatismus eingesetzt werden.

Zu einer Korrektur einer Fehlsichtigkeit (Kurzsichtigkeit, Weitsichtigkeit) werden sphärische Probegläser eingesetzt, während zu einer Korrektur eines Astigmatismus (Hornhautverkrümmung) bzw. einer Winkelfehlsichtigkeit zylindrische bzw. prismatische Probegläser Anwendung finden. Demnach kann jedes Probeglas durch zumindest einen entsprechenden optischen Parameter, insbesondere durch eine Sphäre, einen Zylinder oder ein Prisma, klassifiziert bzw. charakterisiert werden. Das Probeglas kann dann vollständig durch eine Angabe eines numerischen Wertes des optischen Parameters charakterisiert werden. So kann es sich bei einem Probeglas beispielsweise um ein sphärisches Probeglas mit einem numerischen Wert von 1,5 (Dioptrien) handeln. Zu einer besseren Unterscheidbarkeit der Probegläser weisen die Probegläser regelmäßig einen Aufdruck auf, welcher den numerischen Wert anzeigt. Oftmals ist dieser Aufdruck auf einem Griff eines Rahmens des Probeglases aufgebracht. Bei dem Überprüfen der Augen des Probanden können dann solange verschiedene Probegläser in die Glasaufnahmen gesteckt werden, wobei der Proband nach jedem Einsteckvorgang nach einem Ergebnis gefragt wird, bis ein Probeglas bzw. eine Kombination von Probegläser gefunden wird, das bzw. die eine Erkennung der Sehprüfzeichen durch die Augen des Probanden subjektiv optimiert. Nachteilig ist hier jedoch, dass die numerischen Werte für die eingesteckten Probegläser von der Bedienperson alle händisch dokumentiert werden müssen. Dies ist zum einen zeitaufwendig und zum anderen anfällig für Fehler, insbesondere für Abschreibfehler.

Der vorliegenden Erfindung liegt daher die Aufgabe zu Grunde, ein Verfahren zum Überprüfen von Augen eines Probanden mit einem Sehprüfsystem sowie ein Sehprüfsystem vorzuschlagen, mittels dessen eine Durchführung eines Sehtest einfacher erfolgen kann.

Diese Aufgabe wird durch ein Verfahren mit den Merkmalen des Anspruchs 1 und ein Sehprüfsystem mit den Merkmalen des Anspruchs 8 gelöst.

Bei dem erfindungsgemäßen Verfahren zum Überprüfen von Augen eines Probanden mit einem Sehprüfsystem wird vor den Augen eine Messbrille des Sehprüfsystems mit zumindest einem Probeglas angeordnet, wobei mittels einer Kamera einer Kameravorrichtung eines Anzeigegeräts des Sehprüfsystems zumindest ein numerischer Wert eines das Probeglas charakterisieren optischen Parameters erfasst wird, wobei mittels einer Steuervorrichtung des Sehprüfsystems das Anzeigegerät gesteuert wird, wobei der numerische Wert von der Steuervorrichtung mittels Bildverarbeitung erkannt wird, wobei der numerische Wert für eine Sphäre und/oder einen Zylinder und/oder eine Achse und/oder ein Prisma erkannt wird.

Demzufolge weist das Anzeigegerät eine Kameravorrichtung mit einer Kamera auf, mittels derer ein numerischer Wert eines das Probeglas charakterisieren optischen Parameters erfasst wird. Weiter umfasst das Sehprüfsystem eine Steuervorrichtung, welche das Anzeigegerät steuert und den numerischen Wert des optischen Parameters mittels Bildverarbeitung erkennt. Folglich muss eine Bedienperson die numerischen Werte, welche auf den Probegläsern aufgedruckt sein können, nicht mehr vor bzw. nach jedem Einsteckvorgang manuell aufschreiben bzw. dokumentieren, weil diese Aufgabe nun von der Kamera in Kombination mit der Steuervorrichtung übernommen wird. Somit kann sowohl ein Zeitaufwand des Sehtests als auch eine Fehleranfälligkeit des Sehtest erheblich reduziert werden. Insgesamt ist es damit möglich, den Sehtest noch einfacher durchzuführen.

Erfindungsgemäß wird der numerische Wert für eine Sphäre und/oder einen Zylinder und/oder eine Achse und/oder ein Prisma erkannt. Somit werden alle für einen Brillenpass bzw. ein Brillenrezept relevanten Werte direkt mittels Bildverarbeitung erkannt.

Vorteilhafterweise können zumindest einem Auge des Probanden mittels eines Bildschirms des Anzeigegeräts des Sehprüfsystems Sehprüfzeichen visualisiert werden. Die Erkennung des numerischen Wertes kann unmittelbar nach einem Einsteckvorgang erfolgen, noch bevor die Sehprüfzeichen dargeboten werden. Gleichwohl kann die Erkennung auch während der Visualisierung der Sehprüfzeichen durchgeführt werden oder im Anschluss daran, vor einem etwaigen nachfolgenden weiteren Einsteckvorgang.

Weiter kann die Kamera mittels zumindest eines Eingabeelements des Anzeigegeräts bestätigt werden. Beispielsweise kann das Eingabeelement ein Druckknopf, Drehknopf oder eine berührungssensitive Oberfläche sein, der bzw. die von dem Bediener oder dem Probanden nach einem Einstecken der Probegläser betätigt werden kann. Vor der Betätigung des Eingabeelements kann die Messbrille dabei in eine für eine optische Erfassbarkeit der numerischen Werte durch die Kamera günstige Position verbracht werden. Dies kann dadurch erfolgen, dass der Proband einen Kopf des Probanden, an dem die Messbrille angeordnet sein kann, in die entsprechende Position bringt. Weiter ist es denkbar, dass die Kamera zum Beispiel mittels eines Gelenkarms bewegbar ausgestaltet ist. So ist es dann auch möglich die Kamera derart relativ zu der Messbrille auszurichten, dass der numerische Wert optimal erfasst werden kann. Auch kann das Eingabeelement in ein fernsteuerbares Steuergerät des Sehprüfsystems, beispielsweise in ein Mobiltelefon oder einen Tablet-Computer, integriert sein. Ferner kann das Steuergerät sprachsteuerbar sein, so dass die Betätigung der Kamera durch ein gesprochenes Wort betätigt werden kann. Gleichwohl kann vorgesehen sein, dass die Kamera den numerischen Wert ohne eine zusätzliche Betätigung eines Eingabeelements automatisch erfasst.

In einer konstruktiv vorteilhaften Ausgestaltung der Erfindung kann der numerische Wert von der Steuervorrichtung in einer Datenbank des Sehprüfsystems gespeichert werden. Somit kann der Bediener während bzw. nach dem Sehtest auf eine einfache Weise nachvollziehen, welche Probegläser bzw. Kombinationen von Probegläsern im Rahmen des Sehtests bisher bzw. insgesamt verwendet wurden. Nach jedem Einsteckvorgang kann der Proband dann eine Rückmeldung im Hinblick auf eine Veränderung eines subjektiven Seheindrucks des Probanden geben. Beispielsweise kann der Proband verlauten lassen, dass die Sehprüfzeichen von ihm mittels den gerade in eine Haltereinrichtung bzw. Glashaltevorrichtung der Messbrille eingesteckten Probegläsern nun schärfer wahrgenommen werden, als mittels denjenigen Probegläsern, welche unmittelbar vorher in die Halteeinrichtung eingesteckt waren. Eine Information über die Veränderung des subjektiven Seheindrucks kann dabei mittels eines Eingabeelements des Anzeigegeräts eingegeben und ebenfalls von der Steuervorrichtung in der Datenbank gespeichert werden. Somit kann auf eine einfache Weise dokumentiert werden, welches Probeglas bzw. welche Auswahl an Probegläsern zu einer Verbesserung oder einer Verschlechterung des subjektiven Seheindrucks des Probanden führen bzw. geführt haben. Am Ende des Sehtests können dann alle diejenigen für einen Brillenpass bzw. ein Brillenrezept relevanten numerischen Werte, die mit einem optimierten bzw. schärfsten subjektiven Seheindruck des Probanden verbunden sind, in der Datenbank gespeichert und bei Bedarf zum Beispiel in der Form eines ausgedruckten Brillenrezeptes ausgegeben werden.

Auch kann eine subjektive Refraktion des Auges mittels der Messbrille ermittelt werden. Ferner kann die Messbrille Farbfilter oder Polarisationsfilter aufweisen, die jeweils an eine Farbdarstellung und/oder eine Polarisation des Bildschirms angepasst sind, so dass monokulare oder binokulare Sehtest durchgeführt werden können. Wenn beispielsweise eine Messbrille mit linearer oder zirkularer Polarisation vorhanden ist, kann das Anzeigegerät des Sehprüfsystems so ausgewählt werden, dass dieses übereinstimmend mit der Messbrille polarisiert ist. Eine Korrektur einer Polarisation, beispielsweise mit einer λ-Viertel-Folie, ist somit nicht erforderlich.

In einer besonderen Weiterbildung der Erfindung kann mittels der Kamera oder mittels einer weiteren Kamera der Kameravorrichtung eine Gesichtserkennung des Probanden durchgeführt werden. Vorteilhafterweise kann die Gesichtserkennung vor dem Beginn des eigentlichen Sehtest durchgeführt werden. Die Kamera kann dann erkennen, ob die Augen des Probanden schon zuvor mittels des Sehprüfsystems überprüft wurden und im gegebenen Fall eine entsprechende elektronisch geführte Akte des Probanden öffnen bzw. eine neue Akte anlegen.

Weiter kann das Sehprüfsystem mittels eines sprachsteuerbaren Steuergeräts des Sehprüfsystems gesteuert werden. Somit kann der Proband dem Steuergerät durch ein gesprochenes Wort, beispielsweise "besser" oder "schlechter" mitteilen, ob der subjektive Seheindruck des Probanden mit einer Auswahl an eingesteckten Probegläsern bezogen auf eine unmittelbar vorhergehende getroffene Auswahl an eingesteckten Probegläsern verbessert oder verschlechtert ist.

Das erfindungsgemäße Sehprüfsystem zum Überprüfen von Augen eines Probanden umfasst ein Anzeigegerät, wobei das Anzeigegerät einen Bildschirm umfasst, mittels dessen zumindest einem Auge des Probanden Sehprüfzeichen visualisierbar sind, wobei vor den Augen eine Messbrille des Sehprüfsystems mit zumindest einem Probeglas anordbar ist, wobei das Anzeigegerät eine Kameravorrichtung mit einer Kamera aufweist, mittels derer zumindest ein numerischer Wert eines das Probeglas charakterisierenden optischen Parameters erfassbar ist, wobei das Sehprüfsystem eine Steuervorrichtung aufweist, mittels der das Anzeigegerät steuerbar ist, wobei der numerische Wert von der Steuervorrichtung mittels Bildverarbeitung erkennbar ist. Zu den vorteilhaften Wirkungen des erfindungsgemäßen Sehprüfsystems wird auf die Vorteilsbeschreibung des erfindungsgemäßen Verfahrens verwiesen.

In einer vorteilhaften Variante der Erfindung können der Bildschirm und die Kamera in einem gemeinsamen Gehäuse des Anzeigegeräts angeordnet sein. Das Sehprüfsystem wird dann auch besonders einfach handhabbar, da nicht mehrere miteinander gekoppelte und dennoch voneinander beabstandete Geräte zur Durchführung eines Sehtest und zur Anpassung einer Brille benötigt werden.

Gemäß einer zweckmäßigen konstruktiven Ausgestaltung der Erfindung kann die Kameravorrichtung in eine Verwahrposition in dem Anzeigegerät oder in eine Aufnahmeposition außerhalb des Anzeigegeräts bewegbar ausgebildet sein. Weiter kann die Kameravorrichtung an dem Bildschirm angeordnet sein, so dass die Kamera nach Bedarf in eine Verwahrposition, beispielsweise hinter dem Bildschirm, versenkt oder für eine Kameraaufnahme in eine Aufnahmeposition neben dem Bildschirm verbracht werden kann. Eine Bewegung der Kamera von der Verwahrposition in die Aufnahmeposition und zurück kann durch eine Antriebseinheit der Kameravorrichtung erfolgen. Wenn eine vergleichsweise große Kamera verwendet wird, kann ein Umlenkprisma vorgesehen sein, sodass die Kamera platzsparend hinter dem Bildschirm angeordnet werden kann.

Auch kann vorgesehen sein, dass der Bildschirmhintergrund beleuchtet ist. Dabei kann eine Anpassung einer Bildschirmleuchtdichte des Bildschirms an eine Umgebungsleuchtdichte erfolgen, wobei die Anpassung der Bildschirmleuchtdichte in Abhängigkeit der Umgebungsleuchtdichte proportional erfolgen kann.

Vorteilhafterweise kann das Anzeigegerät ein ortsfestes Ferntest-Anzeigegerät aufweisen, dessen Anzeigeflächengröße für Sehtests mit einem Sehabstand von 3 m bis 10 m, vorzugsweise von 4 m bis 8 m, ausgebildet ist und/oder ein mobil handhabbares Nahtest-Anzeigegerät aufweisen, dessen Anzeigeflächengröße für Sehtest mit einem Sehabstand von 10 cm bis 3 m, vorzugsweise 30 cm bis 1 m, ausgebildet ist. Das Ferntest-Anzeigegerät kann dann zur Darstellung von Sehprüfzeichen zum Testen des Fernsehens und das Nahtest-Anzeigegerät zur Darstellung von Sehprüfzeichen zum Testen des Nahsehens genutzt werden. Das Sehprüfsystem kann entweder das Ferntest-Anzeigegerät oder das Nahtest-Anzeigegerät sowie gegebenenfalls weitere Anzeigegeräte oder auch das Ferntest-Anzeigegerät und das Nahtest-Anzeigegerät und gegebenenfalls weitere Anzeigegeräte aufweisen. Vorzugsweise kann das Ferntest-Anzeigegerät in dem oben genannten Sehabstand relativ zu den Probanden ortsfest aufgestellt oder an einer Wand montiert sein. Wenn der Proband relativ zum Ferntest-Anzeigegerät in einer definierten Sehentfernung zur Durchführung von Sehtests platziert wird, kann die Sehentfernung zu dem Ferntest-Anzeigegerät genau bestimmt werden. Auch kann dann eine Anzeigeflächengröße des Ferntest-Anzeigegeräts um ein vielfaches größer sein, als eine Anzeigenflächengröße des Nahtest-Anzeigegeräts, da gegebenenfalls auf der Anzeigefläche des Ferntest-Anzeigegeräts vergleichsweise größere Sehprüfzeichen dargestellt werden. Dadurch, dass das Nahtest-Anzeigegeräts mobil handhabbar ist, kann dieses in einem nahe zu beliebigen Abstand innerhalb des oben genannten Sehabstands relativ zu den Augen des Probanden von einer Bedienperson oder dem Probanden gehalten oder platziert werden. Entsprechende Sehtests können so in unterschiedlichsten Sehabständen zu dem Nahtest-Anzeigegerät durchgeführt werden. Sowohl das Ferntest-Anzeigegerät als auch das Nahtest-Anzeigegerät können mit einem Steuergerät des Sehprüfsystems von einer Bedienperson ferngesteuert werden. Dabei kann als Steuergerät ein Mobiltelefon, ein Tablet-Computer oder dergleichen vorgesehen sein. Auch kann das Nahtest-Anzeigegerät ein Mobiltelefon, ein Tablet-Computer oder dergleichen sein.

In einer vorteilhaften Variante der Erfindung kann die Messbrille als ein Phoropter ausgebildet sein.

Vorteilhafterweise kann das Anzeigegerät eine Beleuchtungsvorrichtung aufweisen, mittels der die Messbrille und/oder das Auge beleuchtbar sein können. Insbesondere dann, wenn Sehtests bei mesopischen oder skotopischen Lichtbedingungen durchgeführt werden, ist es aufgrund einer verminderten Umgebungshelligkeit schwierig, mit einer Kameravorrichtung Augen eines Probanden zur Durchführung bestimmter Sehtest aufzunehmen. Ebenso kann es sich als schwierig erweisen bei den vorstehend genannten Lichtbedingungen den numerischen Wert des das Probeglas charakterisierenden optischen Parameters mit einer Kameravorrichtung bzw. Kamera zu erfassen. Eine Beleuchtungsvorrichtung des Anzeigegeräts kann hier Abhilfe schaffen. Auch kann vorgesehen sein, die Beleuchtungsvorrichtung in die Messbrille zu integrieren.

Vorzugsweise kann die Beleuchtungsvorrichtung eine Infrarotlichtquelle aufweisen. Mit der Infrarotlichtquelle können die Augen des Probanden bzw. die Messbrille, insbesondere das Probeglas, unabhängig von einer Umgebungsbeleuchtung mit Infrarotlicht beleuchtet und mittels einer entsprechend angepassten Kameravorrichtung aufgenommen werden. Insbesondere kann die Kamera dann eine Infrarotkamera sein. Somit kann der Sehtest dann ohne jegliche Beeinflussung durch eine Umgebungshelligkeit durchgeführt werden. Vorteilhaft kann so auch eine Blendung des Probanden durch eine Beleuchtung der Augen mit Infrarotlicht vermieden werden. Auch kann vorgesehen sein, dass die Beleuchtungsvorrichtung mehrere Infrarotlichtquellen, beispielsweise IR-Leuchtdioden, umfasst. Die Infrarotlichtquelle kann unmittelbar benachbart der Kameravorrichtung angeordnet sein. Auch kann die Infrarotlichtquelle derart an der Messbrille angeordnet sein, dass diese das Probeglas unmittelbar beleuchtet.

Weiter kann das Probeglas einen vorzugsweise aus einem Kunststoff ausgebildeten Rahmen mit einem Griff aufweisen, wobei der Rahmen von einer Halteeinrichtung der Messbrille aufgenommen werden kann. Demzufolge kann ein Glas des Probeglases in dem Rahmen aufgenommen sein und von diesem an einer Berandung des Glases geschützt werden. Die Halteeinrichtung bzw. Glashaltevorrichtung kann mehrere Glasaufnahmen aufweisen, in welche gleichzeitig eine Reihe von Probegläsern bzw. Einstreckgläsern eingesetzt werden können. Die Glasaufnahmen können jeweils aus zumindest zwei oder drei Stegen ausgebildet sein, die Probegläser bzw. Einsteckgläser an deren Umfang haltern können. Dabei können die Glasaufnahmen dergestalt ausgebildet sein, dass die Stege lediglich von den Rahmen der Probegläser kontaktiert werden, um das Glas vor Beschädigungen wie beispielsweise Kratzern zu schützen. Weiter können die Glasaufnahmen jeweils zumindest einen Andruckfinger zum Klemmen eines Probeglases aufweisen. Mittels des Griffs kann das Probeglas auf eine einfache Weise in die Halteeinrichtung eingesteckt bzw. nach einer Benutzung aus dieser entfernt werden. Vorzugsweise werden die Probegläser bei Nichtbenutzung in einem Koffer bzw. einer Tasche aufbewahrt, wobei die Probegläser vorteilhaft derart in dem Koffer gelagert sein können, dass diese von einem Bediener mittels des Griffs entnommen werden können.

In einer vorteilhaften Variante der Erfindung kann der Rahmen einen den numerischen Wert zeigenden Aufdruck aufweisen. Dabei kann der numerische Wert unmittelbar auf ein Material des Rahmens aufgedruckt werden oder aber der Rahmen kann einen Aufkleber bzw. ein Klebeetikett aufweisen, auf welchem der numerische Wert aufgebracht werden kann. Eine Druckfarbe des Aufdrucks kann dabei derart gewählt werden, so dass diese von der Kamera optimal erkannt werden kann. Insbesondere kann der Griff den Aufdruck aufweisen.

Auch kann der Rahmen einen RFID-Transponder und/oder Datencode aufweisen. Demnach kann der numerische Wert des optischen Parameters auch mittels eines Datencodes und/oder mit Hilfe eines RFID-Transponders verschlüsselt werden. Bei einer Verwendung eines RFID-Transponders können auf einem Mikrochip des RFID-Transponders zusätzliche Informationen bzw. Daten abgespeichert sein, beispielsweise wie häufig ein Probeglas in der Vergangenheit bereits eingesetzt wurde. Der Bediener kann auf Grundlage dieser zusätzlichen Informationen bzw. Daten dann insbesondere entscheiden, wann ein Probeglas gegen ein neues Probeglas ersetzt werden muss. Auch kann der Rahmen als Datencode einen Barcode, einen Data-Matrix-Code oder einen QR-Code aufweisen.

Gemäß einer konstruktiv vorteilhaften Ausgestaltung der Erfindung kann das Sehprüfsystem ein Steuergerät zur Steuerung des Anzeigegeräts umfassen. Das Steuergerät kann dabei als ein Fernsteuergerät ausgebildet sein. Beispielsweise kann das Steuergerät ein Mobiletelefon oder ein Tablet Computer sein.

Auch kann die Messbrille eine Vielzahl von Probegläsern aufnehmen. Dies erlaubt eine gleichzeitige Korrektur einer Fehlsichtigkeit (Kurzsichtigkeit, Weitsichtigkeit) und eines Astigmatismus und/oder einer Winkelfehlsichtigkeit.

Ferner kann die Messbrille eine Skala, vorzugsweise zur Anzeige eines numerischen Wertes für eine Achse aufweisen. Eine Einstellung der Achse kann dann durch eine Drehung des Probeglases in einer Glasaufnahme einer Haltereinrichtung der Messbrille erfolgen.

Vorzugsweise kann ein zylindrisches Probeglas in einem Rahmen des Probeglases, vorzugsweise in einem Griff des Rahmens, eine Öffnung aufweisen. Die Öffnung kann dann eine Sicht des Bedieners bzw. der Kamera auf die Skala freigeben, so dass eine Einstellung, insbesondere eines bestimmten numerischen Wertes für die Achse, vorgenommen und der eingestellte numerische Wert von der Kamera erkannt werden kann. Dabei kann ein an die Öffnung angrenzender Abschnitt des Rahmens eine Markierung aufweisen, welcher auf einem Strich der Skala positioniert werden kann.

Weitere vorteilhafte Ausführungsformen des Sehprüfsystems ergeben sich aus den Merkmalsbeschreibungen der auf den Verfahrensanspruch 1 rückbezogenen Unteransprüche.

Nachfolgend werden bevorzugte Ausführungsformen der Erfindung unter Bezugnahme auf die beigefügten Zeichnungen näher erläutert.

Es zeigen:
- **Fig. 1**: eine Vorderansicht eines Anzeigegeräts;
- **Fig. 2**: eine Vorderansicht eines Probeglases in einer Ausführungsform;
- **Fig. 3**: eine Vorderansicht eines Probeglases in einer weiteren Ausführungsform.

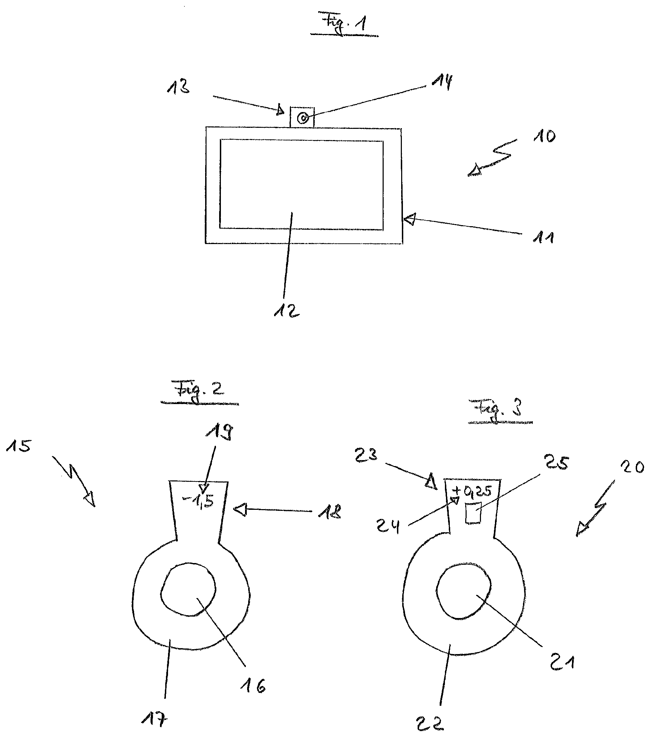

Die **Fig. 1** zeigt ein Sehprüfsystem 10 mit einem Anzeigegerät 11, welches einen hintergrundbeleuchteten Bildschirm 12, in Art eines Fernsehgeräts oder eines Tablet-Computers aufweist. Das Anzeigegerät 11 verfügt über eine Kameravorrichtung 13 mit einer Kamera 14, mittels welcher zumindest ein numerischer Wert eines hier nicht gezeigten Probeglases charakterisierenden optischen Parameters erfasst wird. Weiter umfasst das Sehprüfsystem 10 eine hier ebenfalls nicht gezeigte Steuervorrichtung, mittels derer das Anzeigegerät 11 gesteuert wird, wobei der numerische Wert von der Steuervorrichtung mittels Bildverarbeitung erkannt wird. Mittels des Bildschirms 12 werden einem Probanden Sehprüfzeichen visualisiert, um einen subjektiven Refraktionswert eines Auges des Probanden zu ermitteln. Während eines Sehtests wird dabei eine hier nicht dargestellte Messbrille des Sehprüfsystems 10 mit dem Probeglas vor den Augen des Probanden angeordnet.

Die **Fig. 2** zeigt ein Probeglas 15, welches ein Glas 16 sowie einen Rahmen 17 mit einem Griff 18 umfasst. Weiter ist auf dem Griff 18 ein Aufdruck 19 aufgebracht. Der Aufdruck 19 zeigt hier einen numerischen Wert "-1,5", welcher hier das Probeglas 15 als ein sphärisches Probeglas mit -1,5 Dioptrien charakterisiert.

Die **Fig. 3** zeigt ein Probeglas 20, welches ebenfalls ein Glas 21 sowie einen Rahmen 22 mit einem Griff 23 und einem Aufdruck 24 umfasst. Der Aufdruck 24 zeigt hier einen numerischen Wert "+0,25", welcher das Probeglas 20 als ein zylindrisches Probeglas mit +0,25 Dioptrien charakterisiert. Weiter weist der Griff 23 des Rahmens 22 eine Öffnung 25 auf. Die hier nicht gezeigte Messbrille weist eine Skala zur Anzeige eines numerischen Wertes für eine Achse auf. Wird das Probeglas 20 nun an der Messbrille angeordnet, so kann ein Bediener durch die Öffnung 25 hindurch auf die Skala blicken und eine Einstellung des Wertes für die Achse vornehmen. Für den Fall, dass vor einem Auge des Probanden gleichzeitig ein Probeglas 15 und ein Probeglas 20 zusammen mit der Messbrille angeordnet werden, können die Probegläser 15 und 20 derart verdreht werden, dass der Griff 18 nicht die Öffnung 25 abdeckt und somit eine Sicht eines Bedieners bzw. der Kamera 14 auf den numerischen Wert für die Achse blockiert.

## Patentansprüche

1. Verfahren zum Überprüfen von Augen eines Probanden mit einem Sehprüfsystem (10), wobei vor den Augen eine Messbrille des Sehprüfsystems mit zumindest einem Probeglas (15, 20) angeordnet wird,
**dadurch gekennzeichnet,**
**dass** mittels einer Kamera (14) einer Kameravorrichtung (13) eines Anzeigegeräts (11) des Sehprüfsystems zumindest ein numerischer Wert eines das Probeglas charakterisierenden optischen Parameters erfasst wird, wobei mittels einer Steuervorrichtung des Sehprüfsystems das Anzeigegerät gesteuert wird, wobei der numerische Wert von der Steuervorrichtung mittels Bildverarbeitung erkannt wird, wobei der numerische Wert für eine Sphäre und/oder einen Zylinder und/oder eine Achse und/oder ein Prisma erkannt wird.

2. Verfahren nach Anspruch 1,
**dadurch gekennzeichnet,**
**dass** zumindest einem Auge des Probanden mittels eines Bildschirms (12) des Anzeigegeräts (11) Sehprüfzeichen visualisiert werden.

3. Verfahren nach Anspruch 1 oder 2,
**dadurch gekennzeichnet,**
**dass** die Kamera (14) mittels zumindest eines Eingabeelements des Anzeigegeräts (11) betätigt wird.

4. Verfahren nach einem der Ansprüche 1 bis 3,
**dadurch gekennzeichnet,**
**dass** der numerische Wert von der Steuervorrichtung in einer Datenbank des Sehprüfsystems (10) gespeichert wird.

5. Verfahren nach einem der Ansprüche 1 bis 4,
**dadurch gekennzeichnet,**
**dass** eine subjektive Refraktion des Auges mittels der Messbrille ermittelt wird.

6. Verfahren nach einem der Ansprüche 1 bis 5,
**dadurch gekennzeichnet,**
**dass** mittels der Kamera (14) oder mittels einer weiteren Kamera der Kameravorrichtung (13) eine Gesichtserkennung des Probanden durchgeführt wird.

7. Verfahren nach einem der Ansprüche 1 bis 6,
**dadurch gekennzeichnet,**
**dass** das Sehprüfsystem (10) mittels eines sprachsteuerbaren Steuergeräts des Sehprüfsystems gesteuert wird.

8. Sehprüfsystem (10) zum Überprüfen von Augen eines Probanden, wobei das Sehprüfsystem ein Anzeigegerät (11) umfasst, wobei das Anzeigegerät einen Bildschirm (12) umfasst, mittels dessen zumindest einem Auge des Probanden Sehprüfzeichen visualisierbar sind, wobei vor den Augen eine Messbrille des Sehprüfsystems mit zumindest einem Probeglas (15, 20) anordbar ist,
**dadurch gekennzeichnet,**
**dass** das Anzeigegerät eine Kameravorrichtung (13) mit einer Kamera (14) aufweist, mittels derer zumindest ein numerischer Wert eines das Probeglas charakterisierenden optischen Parameters erfassbar ist, wobei das Sehprüfsystem eine Steuervorrichtung aufweist, mittels der das Anzeigegerät steuerbar ist, wobei der numerische Wert von der Steuervorrichtung mittels Bildverarbeitung erkennbar ist.

9. Sehprüfsystem nach Anspruch 8,
**dadurch gekennzeichnet,**
**dass** der Bildschirm (12) und die Kamera (14) in einem gemeinsamen Gehäuse des Anzeigegeräts (11) angeordnet sind.

10. Sehprüfsystem nach einem der Ansprüche 8 oder 9,
**dadurch gekennzeichnet,**
**dass** die Kameravorrichtung (13) in eine Verwahrposition in dem Anzeigegerät (11) oder in eine Aufnahmeposition außerhalb des Anzeigegeräts bewegbar ausgebildet ist.

11. Sehprüfsystem nach Anspruch 8 bis 10,
**dadurch gekennzeichnet,**
**dass** der Bildschirm (12) hintergrundbeleuchtet ist.

12. Sehprüfsystem nach einem der Ansprüche 8 bis 11,
**dadurch gekennzeichnet,**
**dass** das Anzeigegerät (11) ein ortsfestes Ferntest-Anzeigegerät aufweist, dessen Anzeigeflächengröße für Sehtests mit einem Sehabstand von 3 m bis 10 m, vorzugsweise von 4 m bis 8 m, ausgebildet ist, und/oder ein mobil handhabbares Nahtest-Anzeigegerät aufweist, dessen Anzeigeflächengröße für Sehtests mit einem Sehabstand von 10 cm bis 3 m, vorzugsweise 30 cm bis 1 m, ausgebildet ist.

13. Sehprüfsystem nach einem der Ansprüche 8 bis 12,
**dadurch gekennzeichnet,**
**dass** die Messbrille als ein Phoropter ausgebildet ist.

14. Sehprüfsystem nach einem der Ansprüche 8 bis 13,
**dadurch gekennzeichnet,**
**dass** das Anzeigegerät (11) eine Beleuchtungsvorrichtung aufweist, mittels der die Messbrille und/oder das Auge beleuchtbar ist.

15. Sehprüfsystem nach Anspruch 14,
**dadurch gekennzeichnet,**
**dass** die Beleuchtungsvorrichtung eine Infrarotlichtquelle aufweist.

16. Sehprüfsystem nach einem der Ansprüche 8 bis 15,
**dadurch gekennzeichnet,**
**dass** das Probeglas (15, 20) einen vorzugsweise aus einem Kunststoff ausgebildeten Rahmen (17, 22) mit einem Griff (18, 23) aufweist, wobei der Rahmen von einer Halteeinrichtung der Messbrille aufgenommen werden kann.

17. Sehprüfsystem nach Anspruch 16,
**dadurch gekennzeichnet,**
**dass** der Rahmen (17, 22) einen den numerischen Wert zeigenden Aufdruck (19, 24) aufweist.

18. Sehprüfsystem nach Anspruch 16 oder 17,
**dadurch gekennzeichnet,**
**dass** der Rahmen (17, 22) einen RFID-Transponder und/oder einen Datencode aufweist.

19. Sehprüfsystem nach einem der Ansprüche 8 bis 18,
**dadurch gekennzeichnet,**
**dass** das Sehprüfsystem (10) ein Steuergerät zu Steuerung des Anzeigegeräts (11) umfasst.

20. Sehprüfsystem nach einem der Ansprüche 8 bis 19,
**dadurch gekennzeichnet,**
**dass** die Messbrille eine Vielzahl von Probegläsern (15, 20) aufnehmen kann.

21. Sehprüfsystem nach einem der Ansprüche 8 bis 20,
**dadurch gekennzeichnet,**
**dass** die Messbrille eine Skala, vorzugsweise zur Anzeige eines numerischen Wertes für eine Achse, aufweist.

22. Sehprüfsystem nach Anspruch 21,
**dadurch gekennzeichnet,**
**dass** ein zylindrisches Probeglas (20) in einem Rahmen (22) des Probeglases, vorzugsweise in einem Griff (23) des Rahmens, eine Öffnung (25) aufweist.
